Europäisches Patentamt

European Patent Office

Office européen des brevets

(11) Publication number: 0 200 909
A2

# EUROPEAN PATENT APPLICATION

(21) Application number: 86104244.8

(22) Date of filing: 27.03.86

(51) Int. Cl.4: C12N 15/00 , C07H 21/04 , C12N 1/20 , C12P 21/00 , //C12R1:19

(30) Priority: 03.05.85 SE 8502162

(43) Date of publication of application: 17.12.86 Bulletin 86/46

(84) Designated Contracting States: AT BE CH DE FR GB IT LI LU NL

(71) Applicant: EXCORIM KB
Box 10101
S-220 10 Lund(SE)

(72) Inventor: Björck, Lars
Skogsvägen 20 A
S-240 17 Södra Sandby(SE)
Inventor: Kronvall, Göran
Tornhem Östra Torn
S-223 68 Lund(SE)
Inventor: Lindahl, Gunnar
Magle Lilla Kyrkogata 6
S-223 51 Lund(SE)
Inventor: Kastern, William
Hagedorn Research Laboratory
Copenhagen(SE)

(74) Representative: Boberg, Nils Gunnar Erik
Gambro AB Patent Department Box 10101
S-220 10 Lund(SE)

## (54) Preparation of protein G and/or fragments thereof.

(57) Hybrid-DNA-technique for the manufacture of protein G and/or fragments of protein G with substantially the same properties as protein G insofar as the capability of binding IgG is concerned.

In accordance with the invention a hybrid-DNA-molecule is produced comprising a vector into which has been introduced a DNA-sequence deriving from streptococci which codes for protein G and/or the said fragments thereof.

In accordance with the invention, moreover, a micro-organism is produced belonging, for example, to a strain of E.coli which has been transformed by a hybrid-DNA-molecule in accordance with the above, and a method for preparing the transformed micro-organism.

Finally, a method is provided for the preparation of protein G and/or the said fragments thereof, according to which a micro-organism which has been transformed by the said hybrid-DNA-molecules cultivated in a suitable culture medium and the protein G product is isolated.

Rank Xerox

# PREPARATION OF PROTEIN G AND/OR FRAGMENTS THEREOF

## TECHNICAL FIELD

The present invention concerns the so-called hybrid-DNA-technique and relates among other things to a method in accordance with this technique for the preparation of protein G and/or fragments thereof with the capability of binding immunoglobulin G (IgG).

For more detailed information regarding protein G reference is made to J. Immunol. 133, 969, 1984 together with Swedish patent application No. 8303578-2, applied for 22nd June 1983.

## BACKGROUND ART

The method used up to now for the preparation of protein G is that described in the abovementioned publications and is based on liberating the protein from the surface of streptococci bacteria with the help of proteolytic enzymes, whereafter the protein is isolated.

The method has serious disadvantages. For example, only a part of the protein G molecule will be liberated from the bacteria surface, and even if this part demonstrably is capable of binding IgG, it would naturally be an advantage to isolate the whole molecule. Moreover, this method is restricted to streptococci as a starting material, and, bearing in mind that these are pathogens and difficult to cultivate on a large scale, it would be desirable to find a method which works with other starting materials.

Thanks to the advent of modern so-called DNA-technology (hybrid-DNA-technique) new and better ways have been opened for the preparation of a protein with particularly attractive properties. The technique starts out in that genetic information, which codes for the protein, is transferred with the help of so-called vectors from one cell to another which as a result becomes capable of producing this protein. Such a method is known from the preparation of protein A (cell wall protein of S. aureus) and is described in the two Swedish patent applications no. 8204810-9 and 8204811-7 and the two European patent applications no. 0 107 509 and 0 124 374 respectively.

Compared with protein A, protein G has substantial advantages, especially as a therapeutic agent in the extracorporeal blood treatment in connection with certain autoimmune sicknesses to remove so-called antigen-antibody complexes from the blood. Thus, for example, protein G binds to all IgG-subclasses, whereas protein A lacks the capability of binding the human IgG 3. Furthermore, protein G is a more selective Fc-receptor than protein A, since it does not bind IgA and IgM.

It is an object of the invention, therefore, to provide with the help of the hybrid-DNA-technique, known in itself, a method for the preparation of protein G and/or fragments of protein G with substantially the same properties as protein G insofar as the capability of binding IgG is concerned.

This and other objects have been achieved in accordance with the invention by the method which is specified in the claims and which will be described in detail in the following.

## DISCLOSURE OF INVENTION

In accordance with the invention a hybrid-DNA-molecule is produced comprising a vector into which has been introduced a DNA sequence which codes for a protein capable of being expressed in a micro-organism. The hybrid-DNA-molecule is characterized in that the DNA-sequence codes for protein G and/or fragments of protein G with substantially the same properties as protein G insofar as the capability of binding IgG is concerned.

In accordance with the invention a micro-organism is also produced which has been transformed by a hybrid-DNA-molecule comprising a vector into which has been introduced a DNA-sequence which codes for a protein capable of being expressed in the micro-organism. The micro-organism is characterized in that the DNA sequence codes for protein G and/or fragments of protein G with substantially the same properties as protein G insofar as the capacity of binding IgG is concerned.

In accordance with the invention, moreover, a method is provided for the preparation of protein G and/or fragments of protein G with substantially the same properties as protein G insofar as the capability of binding IgG is concerned. The method involves the incorporation into a micro-organism of a hybrid-DNA-molecule comprising a vector into which has been introduced a DNA-sequence which codes for a protein capable of being expressed in the micro-organism, and is characterized in that the DNA-sequence codes for protein G and/or fragments of protein G with substantially the same properties as protein G insofar as the capability of binding IgG is concerned.

In accordance with the invention a DNA-fragment obtained from a DNA-molecule, which has been isolated from streptococci bacteria, for example group A, C and G streptococci, preferably belonging to group C and/or group G streptococci, is used. In this connection a conventional technique is used including application of known so-called restriction enzymes which cleave the DNA-molecule isolated into suitably large DNA-fragments. The size of the DNA-fragments usable in accordance with the invention may vary and depends among other things on the type of vector which is to be used.

Examples of vectors which can be used for cloning of such protein G-coding DNA-fragments comprise bacterial plasmids (e.g. plasmids from E.coli), phage-DNA (e.g. Phage-lambda or derivatives of lambda, such as EMBL 3 and gt 11, vectors obtained from combinations of plasmids and phage-DNA, yeast plasmids etc. The special selection of vectors is made with a view to the micro-organism (host) used and can readily be made by those versed in the art.

The method of introducing the protein G-coding DNA fragment into the vector used for obtaining the hybrid-DNA-molecule in accordance with the invention is also conventional in this field and is done by using so-called ligating enzymes.

Suitable host organisms, that is to say micro-organisms which can be transformed by the hybrid-DNA-molecule in accordance with the invention, and which consequently become capable of producing the said protein G and/or fragments thereof, comprise gram-negative bacteria - (preferably E.coli), gram-positive bacteria, yeast cells, plant cells etc.

The following examples are merely intended to illustrate a practical mode of procedure for realizing the invention and must not be regarded as limitative in any way.

1. Isolation of chromosomal streptococci-DNA

As a starting material a group G streptococci strain (G148) was selected on the grounds of its high protein G content.

A culture of G148 bacteria in Todd-Hewitt (T-H) medium was allowed to grow over night at 37°C. 3.0 ml of the culture were added to 225 ml T-H and incubated for 3 hours at 37°C. 13.6 ml 10 % cystein and 11.25 ml 0.4 % DL-threonin were added. After incubation for 1 hour at 37°C 125 ml 15 % glycine were added. After a further incubation of 45 minutes at 37°C the cells were washed 3 times in 0.2 M NaOAc and resuspended in 10 ml 0.1M Tris-HCl buffer, pH 8.0, containing 25 % glucose and 10 mM EDTA. 20 mg lysozyme dissolved in 2.0 ml TE buffer (0.01M Tris-HCl, pH 7.4, containing 1.0 mM EDTA) were added. Then incubation took place for one hour at 37°C, whereupon 1.1 ml of 10 % SDS dissolved in TE buffer were added. 500 microlitres of proteinase K (Merch, Darmstadt, West Germany) dissolved in 0.01 M Tris-HCl, pH 7.4, in a concentration of 20 mg/ml and autodigested for 2 hours at 37°C were added. After incubation over night at room temperature 40 ml of TE buffer and 5 ml of 3 M NaOAc, pH 7.4 were added. Then 150 ml absolute alcohol were added, whereupon the solution was incubated at -20°C over night. After centrifuging at 2000 $^x$ G for 30 minutes the supernatant was decanted. The pellet was washed two times with 80 % alcohol and evaporated under nitrogen gas and dissolved in 4 ml TE-buffer, 500 microlitres of RNase-A from bovine pancreas (Sigma, Mo, USA), 2 mg/ml in 0.15 M NaCl heated to 80°C prior to use were added. After incubation for 2 hours at 37°C 200 microlitres of proteinase K (see above) were added. After further incubation over night at room temperature the solution was phenol-extracted twice at room temperature and dialyzed 3 times against TE-buffer. 3 M NaOC was added to a concentration of 0.3 M, whereupon 3 volumes of absolute alcohol were added. After incubation at -20°C over night centrifugation was carried out at 2000 $^x$ 5 for 30 minutes. The pellet was washed twice with 80 % alcohol and evaporated under nitrogen gas. Subsequently the pellet was dissolved in 25 ml TE-buffer. 25 g of caesium chloride (BRL, Bethesda, USA) and 1 ml of ethidium bromide (5 mg/ml) (Sigma, Mo, USA) were added. After centrifugation at 138 000 $^x$ G for 20 hours the DNA-band which in UV-lights has been visualized with ethidium bromide was drawn off. Extraction twice with absolute butanol was followed by dialysis of the bottom phase against the TE-buffer, whereupon NAOAc was added to 0.3M. This was followed by alcohol precipitation with absolute alcohol and washing twice with 80 % alcohol. The pellet was dissolved in distilled water.

Cloning of steptococci-DNA in EMBL 3-Lambda vector

A derivative of phage lambda was used as a vector for the purified streptococci-DNA fragmented by restriction enzymes.

The derivative, EMBL 3, is commercially available (Promega Biotec, WI, USA) and the procedure as to how the phage-DNA and the foreign DNA are prepared and cleaved by restriction enzymes and how the DNA-fragments are bound together - (ligated) has been described in detail in J.Mol. Biol. 170, 827, 1983. In accordance with this known procedure streptococci-DNA-fragments of a size of 10-25 kb (determined by gel electrophoresis in 1 % agarose) are incorporated in EMBL 3-DNA. lambda-streptococcal-hybrid-DNA-molecules were enclosed in the protein shell which marks an intact phage lambda capable of infection. A description of how this takes place may be found in the Promega Biotecs catalogue (entitled Promega Biotec, Molecular Biologicals). The end result was phage lambda containing lambda-DNA-molecules into which fragments of varying size of streptococci-DNA has been incorporated.

Infection of E.coli bacteria by EMBL 3 lambda-phages containing streptococci-DNA

As host cell for the lambda-streptococci-hybrid-DNA-molecules produced were selected the two E.coli-strains NM 538 and NM 539. These strains can be infected by EMBL 3-phages and are described in detail in J.Mol.Biol. 170, 827, 1983. The strains were obtained from Promega Biotec, WI, USA. A bacteria colony of each strain was innoculated over night in 20 ml Lb-medium (10g NaCl, 10g Difco tryptone, 5g Difco yeast extract, pH adjusted to 7.4 with 5M NaOH) containing maltose. 100 microlitres phage solution (according to 2 above) were blended with 200 microlitres NM 538 and NM 539 bacteria. Thereafter they were incubated for 30 minutes at 37°C. To both tubes 3 ml LB maltose medium containing 10 mg $MgCl_2$ and 0.7 % agarose was added. The solutions - (60°C) were poured out onto LB plates (made of LB medium containing 1.5 % agar), a thin agarose layer being formed when the agarose solidified. The plates were incubated over night at 37°C.

If the bacteria are not infected a uniform mat of cells would cover the plates, whereas in the event of infection holes, so-called plaques, are formed in the cell mat. These plaques are formed owing to the infected coli bacteria being lysated by the phages, which involves the cells falling apart and phages and other intracellular material flowing out.

On our plates 500-2000 plaques/plate appeared. On the assumption of the EMBL 3-phages containing streptococci-DNA coding for protein G and on the assumption that this DNA can be replicated and expressed in E.coli, production of protein G too can take place in the infected bacteria and it should be possible to detect protein G in corresponding plaques when the bacteria burst owing to the phage infection and release their content.

Bearing in mind that the streptococci-DNA-fragments which have been incorporated in the EMBL 3-DNA-molecules are approx. 20 kb sized and the streptococci genome amounts to several thousand kB, statistically only a small number of the plaques should contain protein G.

Identification of plaques containing protein G

For the detection of protein G in the said plaques the capability of the molecule to bind IgG was utilized. Nitrocellulose filters (BA 85 Membranfilter, Schleicher und Schuell, Dassel, West Germany) were placed on top of the plates with the plaques for 10 minutes at room temperature. Proteins, phages and other material from the plates are then absorbed into the filters. In order to block the continued facility of the filters to bind proteins the filters were placed into a special so-called blocking buffer (31.1 ml 5M NaCl, 10.0 ml 1M Tris HCl, pH 7.4, 50 ml Tween 20, distilled water added to 1 litre and 0.25 g gelatine). The buffer was changed 4 times every tenth minute (100 ml buffer each time), whereupon the filters were placed in 100 ml blocking buffer, containing 200 microlitres peroxidase-marked rabbit IgG (DAKO-Immunoglobulins A/S, Denmark) and treated on the shaking table for 30 minutes at room temperature. If protein G has been adsorbed by the filters the peroxidase-marked IgG-antibodies will be bound to the filters. The filters were washed again 4 times with blocking buffer and were then placed into a fresh colouring solution (4 ml 1 % 3-amino-9-ethyl-carbazol + 100 ml 50 mM NaOAc, pH 5.1 + 10 microlitres $H_2O_2$), whereupon the peroxidase-marked IgG-antibodies which have been bound to the filters, should emerge as brightly shining red spots. On the filter under test a total of 26 red, shining pin-head-sized spots, corresponding to a total of approx. 6000 plaques were observed. As a positive check a filter was used onto which had been applied 0.25 micrograms of pure protein G - (isolated according to J. Immunol. 133, 969, 1984) in one microlitre of distilled water, whereupon the filter had been treated in the same manner as the others. A brightly shining red spot appeared where the protein G had been applied whilst the remainder of the filter was white.

From the original plates the plaques corresponding to the ten strongest-coloured red spots on the filter were then picked up with a Pasteur pipette. This material was washed into ten tubes, each containing 1 ml SM-buffer (20 mM Tris-HCl, pH 8.0, 0.1M NaCl, 10 mM $MgCl_2$ and 0.1% gelatine) and was used to infect again NM 538 and NM 539 bacteria. The whole procedure was repeated, but in this case on eight of the ten plates > 50 % of the plaques were red, that is to say containing protein G. New plaques were picked and the whole procedure repeated 2 more times, whereafter red spots found on three of the plates corresponded to the total of plaques. As a result therefore we obtained EMBL 3-phage preparations consisting of phages which all contained protein-G-genes. One of these preparations (named lambda PG4B) has been deposited on 29th March 1985 at ATCC under number 40176.

5. Production of protein G through infection of E.coli-bacteria by hybrid-DNA-molecules consisting of streptococci-DNA and EMBL 3-phage-DNA

The EMBL 3-phages containing protein G-genes prepared and purified according to the above were used for infecting NM 538 and NM 539 bacteria. A colony of NM 538 and a colony of NM 539 were added respectively to two beakers each containing 100 ml LB maltose medium with 10 mM $MgCl_2$ Furthermore 100 microlitres of EMBL 3-phages containing protein G-genes were added to each of the two beakers. The beakers were incubated for 8 hours whilst being shaken at 37°C. Bacteria and bacteria residues were centrifuged down (10000 $^x$ G for 20 minutes). The supernatant was analyzed in respect of protein G with the help of peroxidase-marked antibodies and with pure protein G as a standard. In the supernatant 5-10 nanograms of protein G/ml were detected. This protein G has been analyzed by the Western blot procedure making use of radioactively marked IgG as a probe and the molecular weight was determined at approx. 60.000.

Cloning of protein G-genes in lambda gt 11 -vector

EMBL 3-DNA containing the 20 kb sized protein G-DNA-fragment was isolated from lytic plaques. EMBL 3-DNA which does not contain any foreign DNA cannot be cleaved by the restriction enzyme EcoRI (lacking EcoRI-site). On the other hand there was a possibility that the 20kb sized fragment could be cleaved by this enzyme. If EcoRi cleaves outside the protein G-genes this would allow us to isolate a smaller DNA-fragment containing protein G-genes. The EcoRI-treated DNA-material was ligated thereafter with DNA from lambda phage gt 11. The ligation and subsequent construction of an intact phage particle was done by the same method as described above for EMBL 3. Subsequently, the E.coli strain Y1090 (ATCC Rockville, Maryland, USA) was infected with these lambda gt 11-phages, likewise in accordance with the procedure described for EMBL 3. In the analysis of the plates it was found that all plaques contained protein G. From the plates lambda gt 11 phages were purified in conventional manner. Phage-DNA was isolated and the streptococci-DNA which had been ligated with phage-DNA was cut out again with EcoRI. Analysis by electrophoresis showed that this DNA-fragment containing protein G-genes is 4.4 kb sized. Hybrid-DNA-molecules constructed from lambda gt 11-DNA and this 4.4 kb sized DNA fragment were also used in order to infect another E.coli strain (Y1089 ATCC). This strain makes possible an incorporation of the hybrid-DNA-molecule into the E.coli genome when stable lysogens have been achieved.

To sum up it can be said that it is possible in accordance with the invention to incorporate protein G-genes into bacteriophage-DNA and to infect with the hybrid-DNA-molecules constructed E.coli-bacteria which as a result are enabled to produce protein G.

**Claims**

1. A hybrid-DNA-molecule comprising a vector into which is introduced a DNA sequence which codes for a protein capable of being expressed in a micro-organism, **characterised** in that the DNA-sequence codes for protein G and/or fragments of protein G with substantially the same properties as protein G insofar as the capability of binding to IgG is concerned.

2. A hybrid-DNA-molecule in accordance with claim 1, **characterised** in that the said DNA-sequence derives from streptococci.

3. A micro-organism, **characterised** in that it is transformed by a hybrid-DNA-molecule in accordance with claim 1 or 2.

4. A micro-organism in accordance with claim 3, **characterised** in that it is selected among gram-negative bacteria, gram-positive bacteria, yeast cells and plant cells.

5. A micro-organism in accordance with claim 3 or 4, **characterised** in that it is a strain of E.coli.

6. A method for the preparation of a transformed micro-organism in accordance with anyone of claims 3-5, **characterised** in that micro-organisms are transformed in a manner known in itself by a hybrid-DNA-molecule in accordance with anyone of claims 1-2.

7. A method for the preparation of protein G and/or fragments of protein G with substantially the same properties as protein G insofar as the capability of binding to IgG is concerned, **characterised** in that a transformed micro-organism in accordance with anyone of claims 3-5 is cultivated in a suitable culture medium and the protein G product formed is isolated.